# EUROPEAN PATENT SPECIFICATION

(11) **EP 0 722 745 B1**
(45) Date of publication and mention of the grant of the patent: **29.12.1999**
(21) Application number: 96300441.1
(22) Date of filing: 23.01.1996
(51) Int. Cl.: A61M 5/152

(54) **Apparatus for supplying fluid**
Flüssigkeitszuführvorrichtung
Dispositif d'alimentation d'un fluide

(30) Priority: 24.01.1995 JP 889095
(43) Date of publication of application: 24.07.1996
(73) Proprietor: AUBEX CORPORATION, Sumida-ku Tokyo 130 (JP); Opto Tech Co., Ltd., Kohto-ku, Tokyo (JP)
(72) Inventor: Kanai, Masahiro, Sumida-ku, Tokyo (JP); Sato, Hideya, Kohto-ku, Tokyo (JP)
(74) Representative: Jackson, Peter Arthur

(56) References cited:
- WO-A-88/03819
- WO-A-92/06721
- DE-A- 3 940 442
- US-A- 5 167 631
- US-A- 5 360 411

## Description

The present invention relates to an apparatus for supplying fluid which continuously supplies fluid contained within a rubber state elastic film of a tube form by the contracting power of the rubber state elastic film. The present invention relates to an apparatus for supplying fluid used for continuously supplying a living body such as , a human body, an animal, and so on with medical fluid, a transfusion and so on, or for continuously supplying plants with water, nutrients (fluid), medical fluid (an insecticide fluid) and so on, and further, for continuously supplying a fish aquarium with medical fluid such as an antibiotic, or the like, bait (fluid), or nutrients (fluid) for water plants.

Conventionally, as an apparatus for introducing medical fluid which continuously injects medical fluid into a human body in small amounts, an apparatus wherein a medical fluid inflow portion and a medical fluid outflow portion are provided on opposite sides of a case (Japanese Patent Application Laid-open No. 56-102252 official report and so on) is known.

The above-described apparatus is provided with and has a protection case of a cylindrical form, a balloon consisting of an elastic material, sealed within this protection case and containing medical fluid therein, the inflow portion provided on the surface side of one end of the above-described protection case introducing the medical fluid into the balloon, an outflow prevention means such as , for example, a partition wall provided at this inflow portion consisting of soft rubber capable of being sealed, the outflow portion provided on the surface side of the other end of the above-described protection case, through which medical fluid flows out from within the above-described balloon, an introduction tube connected to this outflow portion, and an injector needle connected to the forward end of this introduction tube.

For use, after inserting the forward end of a syringe (injector) containing medical fluid into the inflow portion, the medical fluid within the syringe is ejected into the balloon expanding the balloon. The medical fluid contained within the balloon is prevented from flowing out of the inflow portion by the outflow prevention means, and is continuously injected into a human body in a small amount through the introduction tube by the contracting power of the balloon.

The above-described arrangement, wherein the medical fluid inflow portion and the medical fluid outflow portion are provided at the surface sides of both ends (opposite sides) of the protection case, has the disadvantages of being difficult to hold, and inconvenient to carry, since the medical fluid inflow portion and the medical fluid outflow portion protrude from both sides of the protection case, for example, when a patient having the medical fluid injection holds the protection case within his pocket so as to move easily. In addition, the former apparatus, wherein the medical fluid inflow portion and the medical fluid outflow portion are on the surface sides of both ends of the protection case, has the disadvantages of being difficult to confirm safe operation and being difficult to handle conveniently.

Then, as an apparatus which can eliminate these disadvantages, an apparatus wherein the medical fluid inflow portion and the medical fluid outflow portion are provided on the same side of the case (Japanese Patent Application Laid-open No. 6-121835 official report which the present applicant previously proposed) are known.

This apparatus is provided with and has a protection case of cylindrical form, the balloon consisting of an elastic material, sealed within this protection case, and containing medical fluid therein, the inflow and outflow tubes inserted into the above-described balloon through the surface of one end of the above-described protection case and having a check valve at the side of the outer end portion side, the tube (flow control means) formed from thermoplastic resin, connected to this inflow and outflow tube, and having a noncircular form of a sectional opening hole and a predetermined length, the injector needle provided at the forward end of this tube.

For use, after inserting the forward end of a syringe (injector) containing medical fluid into the inflow and outflow tube, press out the medical fluid within the syringe. Then, the medical fluid is introduced into the balloon through the check valve, expanding the balloon. After the medical fluid has been introduced, extract the forward end of the syringe from the inflow and outflow tube, and then after inserting the forward end of the tube formed from thermoplastic resin into the inflow and outflow tube to open the check valve, opening a cock provided at the midway of the tube. Then, the medical fluid contained within the balloon is injected into a human body in a small amount through the tube by the contracting power of the balloon.

This arrangement, wherein the inflow and outflow of medical fluid can be conducted on the same side, does not have the disadvantages of the former apparatus, however, this arrangement has the disadvantage of being unable to introduce an additional medical fluid therein while continuously injecting medical fluid into the patient, since the inflow and outflow of the medical fluid are conducted through one inflow and outflow tube by switching the direction of the flow.

In both of the above-described prior arts, wherein the sectional forms of the protection cases are circular, the balloons contact the entire range of the inner circumference walls of the protection cases, when the balloons expand from containing the medical fluid. This not only creates resistance when the balloon expands, but since the square area contacting the protection case is large, fluctuations in its contracting power are produced; therefore both apparatuses have the disadvantage that the amount of medical fluid introduced is not stabilized.

The protection case is provided with an air hole therein, in order to permit air to flow into the case and flow out of the case, with the expansion and contraction of the balloon. However, when the balloon contacts the entire range of the inner circumference wall of the protection case, the space in the expansion direction of the balloon within the case is blocked, and further expansion of the balloon is restricted, so that the position where the air hole is attached is provided on the case on the side in the direction of the expansion of the balloon. That is to say, there is a disadvantage that the position of attaching the air hole is limited.

The object of the present invention is to provide an apparatus for supplying fluid which eliminates the above-described conventional disadvantages, is convenient to hold and carry, and can introduce additional fluid, while continuing to supply fluid.

Another object of the present invention is to provide an apparatus for supplying fluid which can be arranged less expensively with the number of the parts being small, and in addition can improve the durability and the handling.

Further, another object of the present invention is to provide an apparatus for supplying fluid wherein the handling is easy, fluid is continuously supplied accurately, and the position where the air hole is attached is not limited.

WO 88/03819 discloses an apparatus for supplying fluid comprising a protection case, a tubular rubber elastic film which is received in the protection case and is arranged to contain fluid, and a fluid supplying tube through which fluid contained within said film is supplied by the contracting power of the film, wherein said protection case is arranged as a cylinder body having a bottom and a lid body engaged in an open end of the cylinder body, and wherein said protection case is provided in the lid body with an inflow fluid hole through which the fluid is introduced into the film, an outflow fluid hole through which the fluid flows into said supplying tube, and a fluid introduction tube inserted into said film, which has an outer diameter and a length virtually equal to an inner diameter and a length of the film when contracted and is provided with slits on a circumference surface thereof by which the inside and the outside of the fluid introduction tube are in fluid communication with each other.

The present invention is characterised in that an inside end of said inflow fluid hole and said outflow fluid hole are joined so that both of said fluid holes communicate with said fluid introduction tube and said rubber state elastic film through said slits; in that an outside end of said inflow fluid hole and said outflow fluid hole extend in a direction away from each other relative to a main axis line of said fluid introduction tube in a Y-shape; and in that a check valve which permits the inflow of the fluid and prevents the outflow of fluid is provided at the inflow fluid hole.

By this arrangement, when the fluid is introduced into the tubular film through the inflow fluid hole and the fluid introduction tube, the fluid introduced into the tubular film is supplied through the outflow fluid hole and the fluid supplying tube by the contracting power of the elastic film. In this regards, the inflow fluid hole and the outflow fluid hole are provided in positions close to each other, that is to say, there is no need to provide the inflow fluid hole and the outflow fluid hole on the surface of both ends of the protection case; therefore the present invention is easy to hold and convenient to carry. Further, the present invention is provided with the inflow fluid hole and the outflow fluid hole; therefore additional fluid can be introduced while continuously supplying fluid.

The apparatus for supplying fluid of the present invention is arranged so that the above-described inflow fluid hole, the above-described outflow fluid hole, and the fluid introduction tube which is inserted into the above-described tubular film has an outer diameter and a length virtually equal to an inner diameter and the length of the tubular film when contracting and has slits on a circumference surface by which an inner portion is communicated with the above-described inflow fluid hole and outflow fluid hole, communicating the inside and the outside, are provided as one body.

By this arrangement, the inflow fluid hole, the outflow fluid hole, and the fluid introduction tube are formed as one body; therefore the number of the parts can be reduced. This means that the amount of assembly work can be reduced, so that the cost can be reduced.

The fluid introduction tube is inserted into the tubular film and has an outer diameter and the length virtually equal to the inner diameter and the length of the tubular film when contracting, as well as slits on the circumference surface by which the inner portions are communicated with the above-described inflow fluid hole and outflow fluid hole, communicating the inside and the outside, and so the fluid supplied from the inflow fluid hole is introduced from the forward end and the slits on the circumference surface of the fluid introduction tube into the tubular film, so that the load against the tubular film does not become partial; therefore the durability of the tubular film can be improved. In addition, the tubular film is contracted to be in a state that closely contacts an outer surface of the fluid introduction tube; therefore the amount of fluid remaining is small when the supply is finished.

In the apparatus for supplying fluid of the above-described arrangement, the film may be made to be a tube having a bottom with one end open, and the open portion of one end may be fixed at a base portion of the above-described fluid introduction tube with the fixing tools such as a pinch and so on. In doing so, the assembly can be conducted by inserting the open portion of one end of the tubular film into the fluid introduction tube and by fixing one end of the tubular film at the base of the fluid introduction tube with the fixing tools such as a pinch and so on; therefore the assembly can be easily conducted.

As the protection case is arranged by a cylinder body of the cylindrical form having a bottom, and a lid body engaged in an open end of this cylinder body, and as the inflow fluid hole, outflow fluid hole, and the fluid introduction tube are formed at the lid body in a Y-shaped form and to be one body, the assembly and disassembly can be easily conducted. In addition, since the inflow fluid hole and the outflow fluid hole are closely provided in a V-shaped form, the introduction and the supply of fluid can be conducted without hindrance, even when the inflow fluid hole and the outflow fluid hole are closely positioned; therefore the entire body can be made compact. Further, the introduction operation of the fluid can be conducted by holding the neck portions of the inflow fluid hole and outflow fluid hole between ones fingers; therefore the handling is easy.

In this case, as for the form of the cylinder body, the sectional surface form in the direction which vertically crosses the axial direction of the fluid introduction tube is desired to be an elliptic form, and the bottom portion is desired to be a curved convex surface form. If the sectional form is elliptical, the area where the tubular film, expanded by the fluid introduced, contacts an inner wall of the cylinder body can be made smaller compared to the protection case with a circular sectional form, and the air flow within the protection case can be secured. Accordingly, the fluid can be continuously supplied accurately, and the position where an air hole is attached is not limited. In this case, if a water repellent air filter is attached in the air hole, the fluid can be prevented from flowing from the protection case, while the air inside and outside of the protection case is circulated. In addition, the protection case does not roll down, even if the protection case is put on a tilted surface; therefore handling is easy. If the bottom portion is made to be a curved convex surface form, the protection case can be made more compact by as much as the edge portion of the bottom portion which becomes a curved surface, compared to the simple cylindrical form, and the protection case is easily held in a pocket.

Further, the cylinder body is desired to be formed of transparent material. In this case, a scale showing the amount of fluid contained within the tubular film from the position of the forward end portion of the rubber state elastic film is desired to be provided on an outer circumference of the cylinder body. In this way, the amount of the fluid contained within the rubber state elastic film is seen through the cylinder body and known from the scale.

A spring of a spiral form, which is positioned at the outer circumference of the tubular film and extends with the expansion of the tubular film, may be engaged in the lid body. In this case, the spring is desired to be arranged so as to be coiled in a spiral state wherein the diameter becomes narrower as it proceeds downwards, with the top end being engaged in the above-described lid body, and with the lowest end abutting the forward end of the above-described medical fluid introduction tube with the above-described tubular film between them. In this way, the amount of fluid contained within the tubular film is accurately known from the relation between the scale and the position to which the spring extends. In addition, the square area where the tubular film contacts the protection case when the tubular film expands can be made small by the function of the spring, and the contraction of the tubular film is surely conducted; therefore the amount of the fluid remaining, when the supply is finished, is small.

At the inflow fluid hole, a check valve, which permits the inflow of the above-described fluid and prevents the outflow of the above-described fluid, is provided. The check valve may be provided with a valve sheath which is laid within the inflow hole and has a valve seat at the midway position therein, and a valve spindle which is contained within the valve sheath so as to be movable, opening and closing the valve seat. In this case, if a cap can be engaged in the outer end of the valve sheath so as to be attachable and removable, alien substance can be prevented from entering.

If a fluid supplying tube is arranged by a thermoplastic resin tube having the noncircular sectional open form and the predetermined length, the flow amount of the fluid is controlled to be a fixed amount by means of the tube having the introduction function and the flow control function.

In the accompanying drawings:
Fig. 1 is a perspective view illustrating a preferable embodiment of an apparatus for supplying fluid related to the present invention;
Fig. 2 is a sectional view of the above-described embodiment;
Fig. 3 is a sectional view of a protection case of the above-described embodiment;
Fig. 4 is a sectional view of a water repellent air filter of the above-described embodiment;
Fig. 5 (A), Fig. 5 (B), Fig. 5 (C), Fig. 5 (D), and Fig. 5 (E) are sectional views illustrating different sectional forms of a tube used for the apparatus of the above-described embodiment;
Fig. 6 is a view illustrating the state when medical fluid is introduced into the apparatus of the above-described embodiment; and
Fig. 7 is a sectional view illustrating the relationship between the protection case and a tubular film, when medical fluid is introduced into the apparatus of the above-described embodiment.

A preferable embodiment of the present invention will be described with reference to the drawings.

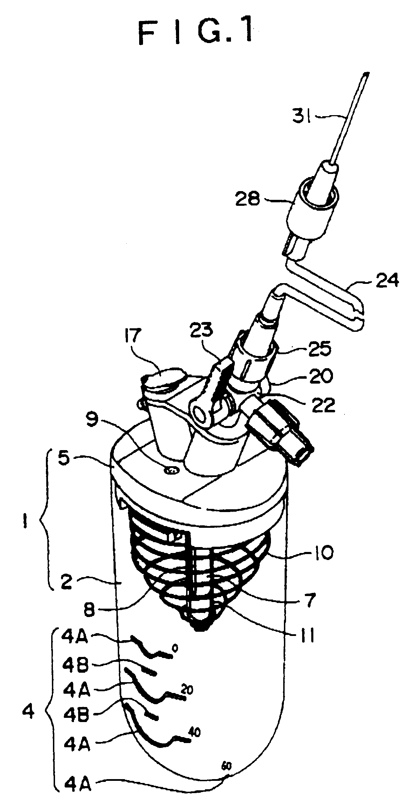

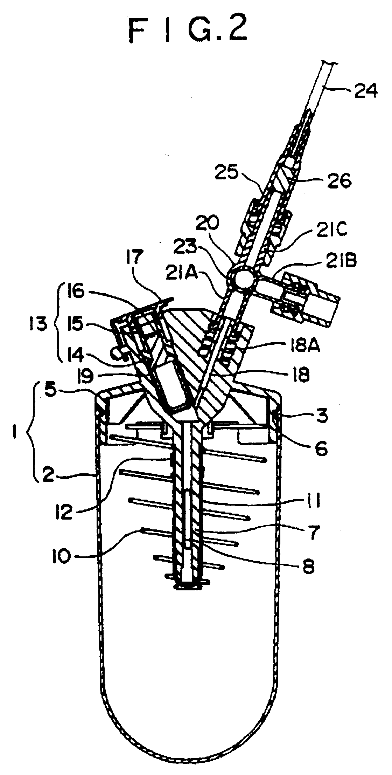

The present embodiment is an embodiment which is applied to an apparatus for injecting medical fluid into a human body. In Fig. 1 and Fig. 2, 1 is a protective case consisting of a cylinder body 2 which is made in a cylindrical form having a bottom of transparent material such as plastic, glass, and of a lid body 5 which is engaged in the open end of the above-described cylinder body 2 and is formed from such material as polypropylene.

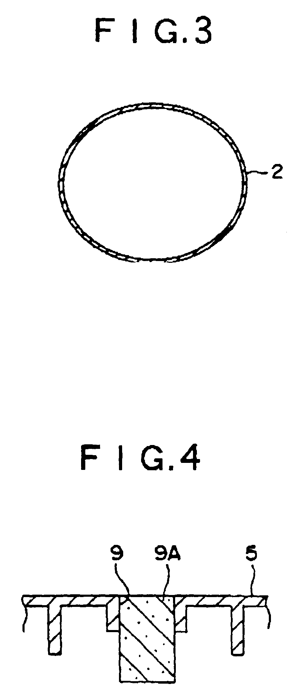

The above-described cylinder body 2 is formed to have a cross sectional form as in Fig. 2 and is formed having a noncircular form, and specifically in this embodiment the cylinder body 2 is formed to be an elliptic form (refer to Fig. 3), formed to be a cylindrical form having a bottom of a curved convex surface, with a projection 3 being provided at the inner circumference wall of the open end side, while a scale 4 is provided at the outer circumference wall. As the scale 4, even-numbered divisions 4A which are 0, 20, 40, and 60 showing the amount of medical fluid contained in a rubber state elastic film 11 described below in units of cc, and odd-numbered divisions 4B which are 10, 30, and 50 positioned between these even-numbered divisions, are provided at the position which is in an up-and-down direction from the center to the bottom portion.

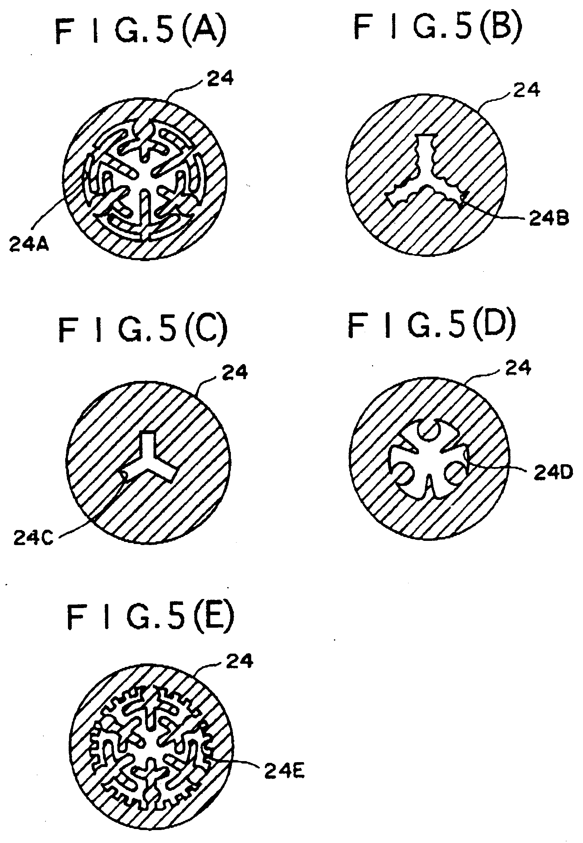

In the above-described lid body 5, formed at the outer circumference wall surface is a concave portion for engagement at 6 in the projection 3 of the above-described cylinder body 2, and a medical fluid introduction tube 7 as a fluid introduction tube of a slender cylindrical form extending towards the inside of the above-described cylinder body 2, and an air hole 9 are respectively provided in a virtual center position of the top wall. The medical fluid introduction tube 7 has both ends open, and has a plurality of slits 8 which communicate the inside and outside, at its circumference surface. As Fig. 4 illustrates, in the air hole 9, attached is a water repellent air filter 9A which circulates gas (air) inside and outside of the protection case 1 preventing the medical fluid from flowing. As the water repellent air filter 9A, for example, a filter made of a repel treated synthetic fiber and of medicine proof quality is preferable.

In the above-described medical fluid introduction tube 7, a rubber state elastic film 11 of tubular form having a bottom with one end being opened is engaged so as to adhere closely, and the open end edge is held by a pinch 12 as a fixing tool. The external diameter and the length of the medical fluid introduction tube 7 is formed to be the size which is virtually equal to the internal diameter and the length of the elastic tube 11 when contracting. In the tube 11, a maximum of 60 cc of medical fluid can be contained. It may be mentioned that in the case of medical fluid for cancer pain, about 20 cc is normally injected a day; therefore medical fluid for virtually three days can be contained. The tube 11 expands as the medical fluid is injected and contained, and a spring 10 which extends as the tube 11 extends is positioned at the outer circumference. The spring 10, consisting of a wire rod of, for example, about a 0.6 to 0.8 mm line diameter, is coiled in a spiral state wherein the diameter becomes narrower as it goes downwards, with the top end being engaged in the above-described lid body 5, and furthermore the lowest end is abutted to the forward end of the medical fluid introduction tube 7 with the tube 11 between them.

It is desirable that the above-described tube 11 is made from a tenacious and highly elastic material of medical proof quality which is not easily damaged by the action of the medical fluid, and transparent or semitransparent material is especially preferable. For example, silicone rubber, and latex rubber currently on the market are preferable. The thickness is about 0.4 mm. The pressure of a 1000 to 7000 mm water column is preferable for the contraction ability when the medical fluid is contained in the rubber state elastic film 11. Normally, a vein of the human body is approximately a 60 nun water column; therefore by increasing the pressure, a patient can be given an injection. When the contraction ability of the rubber state elastic film 11 is less than the 1000 mm water column, controlling become difficult, while with more than that of the 7000 mm water column, it becomes difficult to inject the medical fluid from the syringe to the tube 11 by human power. However, this is not restrictive.

At the top portion (the lid body 5) of the above-described medical fluid introduction tube 7, an inflow hole 19 as an inflow fluid hole through which the medical fluid is injected into the above-described tube 11, and an outflow hole 18 as an outflow fluid hole through which medical fluid contained in the above-described tube 11 is discharged are closely provided in a V-shaped form. That is to say, at the lid body 5, the inflow hole 19, the outflow hole 18, and the medical fluid introduction tube 7 which are communicated with one another are provided in a virtually Y-shaped form to be one body. Within the above-described inflow hole 19, a check valve 13 which permits the flow of the medical fluid into the medical fluid introduction tube 7 from the outside and prevents the flow of the medical fluid to the outside from the medical fluid introduction tube 7 is provided. The check valve 13 is provided with a valve sheath 15 which is laid within the inflow hole 19 and has a valve seat 14 at the midway position therein, and a valve rod 16 which is contained within the valve sheath 15 so as to be movable, opening and closing the valve seat 14, and is of medicine proof quality consisting of silicone rubber. At the outer end portion of the valve sheath 15, a cap 17 can be attached so as to be attachable and removable. A spiral flute 18A which engages a three-way valve 20 so as to be attachable and removable is formed around the above-described outflow hole 18. The three-way valve 20 includes a valve body 22 having three switchover valves 21A, 21B, and 21C, and a cock 23 which changes a flow route.

A connector 25 provided at one end of a thermoplastic tube 24 which doubles as the flow controlling means, is connected to the switchover valve 21C of the above-described three-way valve 20 so as to be attachable and removable. A filter 26 which eliminates dust and so on in the medical fluid is contained within the connector 25. A connector 28 which is similar to the connector 25 is fixed at the other end of the tube 24, and at the forward end of this connector 28, an injector needle 31 as an instrument attached to the human body so as to be attachable and removable. Accordingly, the inside of the rubber tube 11 and the injector needle 31 as the instrument attached to the human body are connected through the thermoplastic tube 24 as means for controlling the flow.

As for the tube used as the thermoplastic tube 24, a single-layered tube is fine, or a tube which is coated, considering the reinforcement and handling, may be fine. As for the material of the tube 24, polypropylene (PP), Polyethylene (PE), polyvinyl acetal (POM). polycarbonate (PC), ABS, polyamide resin, polystyrene (PS), and so on can be used, and transparent material is preferable. For the coating material when coating, flexible material is preferable, a thermoplastic resin elastomer can be used, and a polyolefin (LDPE, LLDPE) type elastomer, a thermoplastic polyurethan elastomer, a soft polyvinyl chloride resin, EVA, and so on can be used.

A sectional form of an opening hole of the tube 24 has a different shaped opening hole, not that of a circular opening hole, and some of the examples are illustrated in Fig. 5 (A), Fig. 5 (B), Fig. (C), Fig. 5 (D), and Fig. 5 (E).

In Fig. 5 (A), Fig. 5 (B), Fig. 5 (C), Fig. 5 (D), and Fig. 5 (E), an opening hole 24A of the tube 24 illustrated in Fig. 5 (A) is made to be the form wherein there are two different kinds of branches each of which has three projections alternately projected towards the center from the inner wall of the round-shaped basic hole.

An opening hole 24B of the tube 24 illustrated in Fig. 5 (B) is made to be the form wherein virtual rectangular openings are extended at equally divided positions of 120 degrees from the center of the tube 24 in the radius direction, and are made to be the virtually Y-shaped form as a whole, with concaves and convexes being formed on the inner wall.

An opening hole 24C illustrated in Fig. 5 (C) is made to be the form wherein the concaves and convexes are eliminated from the inner wall of Fig. 5 (B), and the length in the radius direction of each rectangular opening is shorter than in Fig. 5 (B).

An opening hole 24D illustrated in Fig. 5 (D) is made to be the form wherein three of the slender triangle-shaped and round-shaped projections are alternately projected towards the center from the inner wall of the round-shaped basic hole.

An opening hole 24E illustrated in Fig. 5 (E) is made to be the form wherein the form of the branch shaped projections are changed a little from that of Fig. 5 (A), and concaves and convexes which form a gear with inner cogs are provided on the inner wall of the basic hole of Fig. 5 (A).

The effects of these noncircular opening holes of the tube 24 are remarkable, when the value of the different degree determined by the inner circumference rim dimension of the opening hole/the square root of the square area of the sectional opening hole is more than 7, and the different degrees of each of the above-described opening holes 24A to 24E is made to be large, and more than 7.

For this connection, it may be mentioned that the manufacturing method of the above-described tube 24 in a form of a minute noncircular opening hole is forming by using the dies described in, for example, Japanese Patent Application Laid-open No. 51-21927. That is to say, the tube 24 in a form of the minute noncircular opening hole is obtained, by using monofilament dies which have a number of resin introduction holes within the range virtually equal to the outer diameter of the tube 24, and does not form a hole at the portion corresponding to the opening holes 24A to 24E, by pressing out a molten resin monofilament through this introduction hole, and by uniting a number of monofilaments which are close together. However, the manufacturing method of the tube 24 is not limited to this.

Next, the method for using the preferable embodiment is described

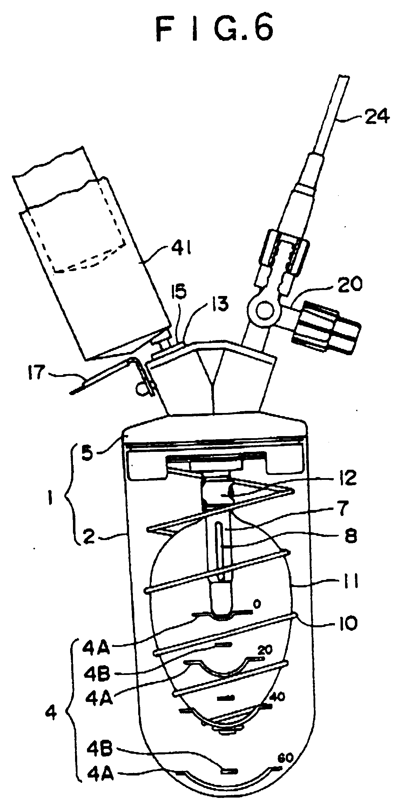

In order to introduce the medical fluid into the rubber tube 11, a cap 17 is removed from the valve sheath 15 of the check valve 13, and the forward end of a syringe 41 containing the medical fluid is inserted into the valve sheath 15, as Fig. 6 illustrates. When the medical fluid in the syringe 41 is pressed out in this state, the medical fluid is introduced into the rubber tube 11 through the check valve 13, expanding the elastic film 11. At this time, the spring 10 extends with the expansion of the rubber tube 11, and therefore the amount of medical fluid contained in the rubber tube 11 can be known from the position in the scale 4 corresponding to the forward end position of the spring 10.

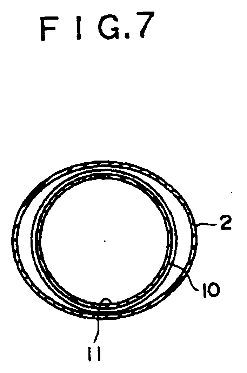

Then, the rubber tube 11 touches the inner circumference wall of the cylinder body 2 of the protection case 1. At this time, since the sectional form of the protection case 1 is formed to be the ellipse form as Fig. 7 illustrates, the square area where the rubber state elastic film 11 touches the protection case 1 can be made small compared to that of the circular form. Since the air flow is secured within the protection case 1, the air within the cylinder body 2 is discharged outside through the water repellent air filter 9A, with the expansion of the rubber tube 11. Accordingly, the medical fluid is continuously, accurately supplied in a small amount, and the position to which the air hole 9 is attached is not limited. After introducing the medical fluid, when the forward end of the syringe 41 is pulled out of the check valve 13, the valve seat 14 of the check valve 13 is closed. Accordingly, the medical fluid within the rubber tube 11 does not leak outside.

Next, after the injector needle 31 is attached to the connector 28 at the forward end of the tube 24, and this injector needle 31 is attached to the human body, when the cock 23 of the three-way valve 20 is opened, the medical fluid is successively introduced into the human body by a very small flow amount through the tube 24. It is mentioned that the present invention is frequently used for the very small flow amount of about 0.8 ml/hr, however, the flow amount can be specified, according to the form and the length of the noncircular opening hole, and the viscosity of the medical fluid, and the flow amount is not limited to 0.8 ml/hr. When all the medical fluid within the rubber state elastic film 11 has been introduced in the human body in this way, the medical fluid is introduced into the rubber tube 11, and the above-described operation is repeated. In order to extract the air within the rubber tube 11, before the injector needle 31 is attached to the human body, it is fine if the protection case 1 is vertically stood with the lid body 5 side up, and the cock 23 is left open until the medical fluid has flown out of the needle point.

The above-described embodiment, wherein the rubber tube 11 is contained in the protection case 1, and the inflow hole 19 which introduces the medical fluid into the rubber tube 11, the outflow hole 18 from which the medical fluid within the rubber tube 11 flows out, and the medical fluid introduction tube 7 are formed in a Y-shaped form to be one body, can reduce the number of the parts. Accordingly, the amount of assembly work can be reduced, so that the cost can be reduced.

This means that the above-described embodiment has no need to provide the inflow hole 19 and outflow hole 18 at the cylinder body 2 side of the protection case 1; therefore, the above-described embodiment is easy to be contained in a pocket or the like, and conveniently carried, if this cylinder body is made to be a lower part. In addition, the bottom portion of the cylinder body 2 side is made to be a curved convex surface form, so that the above-described embodiment can be made compact and easy to be contained in a pocket, reducing the feeling of carrying a foreign object for the patients to whom it is given. Further, the above-described embodiment, which includes the inflow hole 19 and the outflow hole 18, can additionally introduce medical fluid into the rubber tube 11 as it continues the injection of the medical fluid. The protection case 1, which consist of the cylinder body 2 and the lid body 5, is easy to assemble and disassemble.

Since the inflow hole 19 and the outflow hole 18 are provided in the V-shaped form at the lid body 5, the introduction and supply of the medical fluid can be conducted without hindrance, even when the inflow hole 19 and outflow hole 18 are closely positioned; therefore the whole body can be made compact. That is to say, as Fig. 6 illustrates, even when the syringe 41 is inserted into the valve sheath 15 of the check valve 13, the syringe 41 does not interfere with the three-way valve 20, and therefore the introduction and supply of the medical fluid can be conducted without hindrance. Furthermore, the introduction operation of the medical fluid can be conducted by holding the neck portions of the inflow hole 19 and outflow hole 18 between ones fingers; therefore the handling is easy.

The medical fluid introduction tube 7 has the outer diameter and the length virtually equal to the inner diameter and the length of the rubber tube 11 when contracting, as well as the slits on the outer circumference surface by which the inner portion is communicated with the inflow hole 19 and the outflow hole 18, communicating the inside and the outside, and the medical fluid supplied from the inflow hole 19 is introduced into the rubber tube 11 from the forward end and the slits on the outer circumference surface of the medical fluid introduction tube 7, so that the load against the rubber tube 11 does not become partial; therefore the durability of the rubber tube 11 can be improved. In addition, the rubber tube 11 is contracted to be in the state where it closely touches the outer surface of the medical fluid introduction tube 7; therefore the quantity of medical fluid remaining is small, when the supply is finished.

Since the check valve 13 which permits the inflow of the medical fluid and prevents the outflow of the medical fluid is provided at the inflow hole 19, the medical fluid can be introduced into the rubber tube 11 through the check valve 13, and the medical fluid received within the rubber tube 11 is prevented form flowing out of the inflow hole 19, and therefore the actual introduction of the medical fluid can be easily performed. Further, the medical fluid within the rubber tube 11 can be supplied from the tube 24 through the three-way valve 20 connected to the outflow hole 18 therefore the actual supplying of the medical fluid can be easily performed.

With the sectional form of the cylinder body 2 being made in the elliptic form, the square area where the rubber tube 11, expanded by the introduced medical fluid, contacts the inner wall of the cylinder body 2 can be made small, compared to that of the circular sectional form, and the air flow within the protection case 1 can be secured. Accordingly, the fluid can be continuously supplied accurately, and the position where the air hole 9 is provided is not limited. Moreover, the protection case 1 does not roll down even when it is placed on a tilted surface, and so the handling is easy. Of course the medical fluid is prevented from flowing outside, even when the rubber tube 11 is broken, since the rubber tube 11 is sealed within the protection case 1. It is mentioned that in this connection the conventional apparatus for supplying the medical fluid, wherein the sectional form of the protection case is circular, is easy to roll down when placed on the tilted surface, and requires cautions when handling.

The spring 10 of a spiral form, extending with the expansion of the rubber tube 11, is engaged in the lid body 3, and the division 4 is provided at the outer circumference of the cylinder body 2; therefore the amount of the medical fluid contained within the rubber tube 11 can be accurately determined from the relationship between the position to which the spring 10 extends, and the division 4. The square area where the rubber tube 11, when expanded, contacts the protection case 1 can be made small, and the contraction of the rubber tube 11 can be surely done by the function of the spring 10; therefore the amount of the medical fluid remaining is small when the supply is finished.

As the flow control means, the long sized thermoplastic resin tube 24 having the noncircular opening holes 24A to 24 E is used, instead of the short sized tube having the conventional circular opening hole; therefore a precise flow control can be attained by setting the form of the opening hole, and the length of the tube appropriately. When the conventional tube with the circular opening hole is used as the introduction tube, the medical fluid in which dust larger than the inner diameter dimension is floating, or the medical fluid which easily solidifies does not flow at all, since the dust or the solidified substances block the entrance, however, the preferable embodiment uses the tube 24 of the noncircular opening hole form specified, so that the long sides of the noncircular opening holes 24A to 24E are not totally blocked by dust. Accordingly, even if an alien substance and solidified substance are in the medical fluid, the blockade of the opening holes 24A to 24E is more effectively prevented than with the conventional tube of a circular opening hole.

Further, when the conventional circular opening hole tube is used as the introduction tube, the tube is bent, and blocked, because of the weight of a patient lying down, however, when the tube 24 of the noncircular opening hole of the preferable embodiment is used, the tube has resistance to bending, and is not blocked even when the weight falls on it; therefore it is safe. In the medical field where safety is given a priority, the apparatus for injecting the medical fluid which can not be blocked is important.

In addition, the arrangement of the preferable embodiment, wherein the tube 24 possesses both the introduction function and the flow control function, is simpler, compared to the arrangement of the apparatus wherein the conventional tube and the flow control means are combined.

When a conventional small stainless steel tube, or a small glass tube is used for both the introduction function and flow control function, there are many problems of the tube breaking, bending, being too small to be handled and so on, however, the preferable embodiment, which specifies the tube 24 consisting of thermoplastic resin, makes it easy to manufacture the specified forms of the noncircular opening holes 24A to 24E, makes handling easy, and possesses control of a small flow amount and the introduction tube function.

Further, the preferable embodiment, wherein the rubber tube 11 and the tube 24 which is a flow control means are respectively independent, making flow change easy, and able to cope with a wide range of very small flows by selecting and replacing the tube 24 of different forms of the noncircular opening holes and the lengths.

In the above-described embodiment, the sectional form of the cylinder body 2 of the protection case 1 is made to be in the elliptic form, however it is not restrictive. For example, a polygon with more sides than a triangle, the inner circumference surface of the circular hole where projections are formed at intervals of predetermined angles, and so on, either of which are fine, if the form of the inner circumference portion of the protection case 1 is a different formed section other than a circle. The form of the outer circumference portion of the protection case 1 is also not limited to the elliptic form.

In the above-described preferable embodiment, the tube 24 is connected to the outflow hole 18 through the three-way valve 20, but it is not limited to the three-way valve 20, and a one-way opening and closing valve may be fine.

The present invention can be used widely as an apparatus for injecting medical fluid not only for medical treatment in a vein, urology, obstetrics, gynecology, and so on, but also for injecting medical fluid, nutrients, and so on into a living body of an animal, fish and so on.

In addition, the present invention can be used for supplying plants with water, nutrients (fluid), medical fluid (an insecticide fluid), and so on in small amounts. For example, when cultivating vegetables, flowers and trees, in order to supply water, nutrients (fluid), and so on in small amounts, it is fine if only the forward end of the tube 24, or the needle attached to the forward end of the tube 24 is laid under ground around them. In this case, even if the tube 24 is stepped on and bent, the opening hole of the tube 24 is not blocked, so that the supply of the fluid does not stop. When the medical fluid is injected into trees, it is fine if the protection case 1 is hung on the tree by an appropriate means, and the needle of the forward end of the tube 24 is inserted into the tree. This case is not limited to the case when the fluid flows out of the protection case 1 which is hanging in a downward direction, and the medical fluid can be injected into a position higher than the protection case 1.

Further, the present invention can be used in order to supply a fish aquarium with medical fluid such as an antibiotic and so on, bait (fluid), nutrients (fluid) for water plants and so on in small amounts. In this case, it is fine if the forward end of the tube 24 is positioned within the aquarium, without attaching the needle and so on at the forward end of the tube 24.

The apparatus for supplying fluid of the present invention, which is convenient to hold and carry, can introduce an additional fluid as it continues to supply the fluid, can be arranged less expensively with the number of the parts being small, and can improve durability and the handling. The effects of easy handling, and of supplying fluid accurately and continuously without limiting the position at which the air hole is provided are obtained.

## Claims

1. An apparatus for supplying fluid comprising a protection case (1), a tubular rubber elastic film (11) which is received in the protection case and is arranged to contain a fluid, and a fluid supplying tube (24) through which fluid contained within the film is supplied by the contracting power of the film, wherein the protection case is arranged as a cylinder body (2) having a bottom and a lid body (5) engaged in an open end of the cylinder body, and wherein the protection case is provided in the lid body with an inflow fluid hole (19) through which the fluid is introduced into the film, an outflow fluid hole (18) through which the fluid flows into the supplying tube, and a fluid introduction tube (7) inserted into the film, which has an outer diameter and a length virtually equal to an inner diameter and a length of the film when contracted and is provided with slits (8) on a circumference surface thereof by which the inside and the outside of the fluid introduction tube are in fluid communication with each other; characterised in that an inside end of the inflow fluid hole (19) and the outflow fluid hole (18) are joined so that both of the fluid holes communicate with the fluid introduction tube (7) and the rubber state elastic film (11) through the slits (8); in that an outside end of the inflow fluid hole and the outflow fluid hole being extended in a direction away from each other relative to a main axis of the fluid introduction tube in a Y-shape; and in that a check valve (13) which permits the inflow of the fluid and prevents the outflow of fluid is provided at the inflow fluid hole (19).

2. An apparatus according to claim 1, wherein the film is a tube having a bottom with one open end, and an open portion of one end is fixed at a base portion of the above-described fluid introduction tube with fixing tools.

3. An apparatus according to any one of the preceding claims, wherein the cylinder body (2) has an elliptic cross section.

4. An apparatus according to any one of the preceding claims, wherein the bottom of the cylinder body (2) is formed to be a curved convex form.

5. An apparatus according to any one of the preceding claims, wherein the cylinder body (2) is made of transparent material.

6. An apparatus according to any one of the preceding claims, wherein a division (4) showing the amount of the fluid contained within the film (11) from the position of a forward end portion of the film is provided on an outer circumference of the cylinder body (2).

7. An apparatus according to any one of the preceding claims, wherein a spring (10) of a spiral form which is positioned at the outer circumference of the rubber state elastic film (11) and extends with the expansion of the film is engaged in the lid body (5).

8. An apparatus according to claim 7, wherein the spring (10) is coiled in a spiral form where a diameter becomes narrower away from the lid body, with one end being engaged in the lid body (5), and with the other end being abutted to a forward end of the medical fluid introduction tube (7) with the film (11) between them.

9. An apparatus according to any one of the preceding claims, wherein an air hole (9) is formed in the lid body (5) and a water repellent air filter (9A) is attached within the air hole.

10. An apparatus according to any one of the preceding claims, wherein the check valve (13) is provided with a valve sheath (15) which is laid within the inflow fluid hole (19) and has a valve seat (14) at a midway position therein, and a valve rod (16) contained within the valve sheath so as to be free to move, opening and closing the valve seat.

11. An apparatus according to claim 10, wherein a cap (17) is provided at an outer end of the valve sheath (15) so as to be free to move and so as to be engaged.

12. An apparatus according to any one of the preceding claims, wherein the fluid supplying tube (24) is arranged by a thermoplastic resin tube having a noncircular sectional open form and a predetermined length.

## Patentansprüche

1. Eine Vorrichtung zum Zuführen von Flüssigkeit, umfassend ein Schutzgehäuse (1), eine schlauchförmige gummi-elastische Folie (11), die im Schutzgehäuse aufgenommen und so angeordnet ist, daß sie eine Flüssigkeit enthält, sowie ein flüssigkeitsführendes Rohr (24), durch das in der Folie enthaltene Flüssigkeit durch die zusammenziehende Kraft der Folie zugeführt wird, wobei das Schutzgehäuse als zylindrischer Körper (2) angeordnet ist, der einen unteren Teil und einen Deckelkörper (5), der mit einem offenen Ende des zylindrischen Körpers in Eingriff steht, und wobei das Schutzgehäuse im Deckelkörper mit einem Flüssigkeitszufuhrloch (19), durch das die Flüssigkeit in die Folie eingeführt wird, einem Flüssigkeitsausströmloch (18), durch das die Flüssigkeit in das Zufuhrrohr strömt, und einem in die Folie eingesetzten Flüssigkeitszufuhrrohr (7) versehen ist, das einen Außendurchmesser und eine Länge aufweist, die im wesentlichen gleich einem Innendurchmesser bzw. einer Länge der Folie sind, wenn diese kontrahiert ist, und das in einer Umfangsfläche desselben mit Schlitzen (8) versehen ist, durch die die Innenseite und die Außenseite des Flüssigkeitszufuhrrohrs in Flüssigverbindung miteinander stehen, dadurch gekennzeichnet, daß ein innenseitiges Ende des Füssigkeitszufuhrlochs und das Flüssigkeitsausströmloch verbunden sind, so daß beide Flüssigkeitslöcher mit dem Flüssigkeitszufuhrrohr (7) und der gummi-elastischen Folie (11) durch die Schlitze (18) kommunizieren; wobei ein außenseitiges Ende des Flüssigkeitszufuhrlochs und das Flüssigkeitsausströmloch in einer Richtung weg voneinander im Verhältnis zu einer Hauptachse des Flüssigkeitszufuhrrohrs in Y-Form verlängert sind; und indem ein Rückschlagventil (13), das das Zuströmen der Flüssigkeit zuläßt und das Ausströmen der Flüssigkeit verhindert, am Flüssigkeitszufuhrloch (19) angeordnet ist.

2. Eine Vorrichtung gemäß Anspruch 1, in der die Folie einen Schlauch bildet, der einen unteren Teil mit einem offenen Ende hat, und ein offener Teil eines Endes mit Befestigungswerkzeugen an einem Basisteil des oben beschriebenen Flüssigkeitszufuhrrohrs befestigt ist.

3. Eine Vorrichtung gemäß einem beliebigen der vorstehenden Ansprüche, in der der zylindrische Körper (2) einen elliptischen Querschnitt aufweist.

4. Eine Vorrichtung gemäß einem beliebigen der vorstehenden Ansprüche, in der der untere Teil des zylindrischen Körpers (2) in einer konvex gekrümmter Form ausgebildet ist.

5. Eine Vorrichtung gemäß einem beliebigen der vorstehenden Ansprüche, in der der zylindrische Körper (2) aus einem durchsichtigen Material hergestellt ist.

6. Eine Vorrichtung gemäß einem beliebigen der vorstehenden Ansprüche, in der eine Teilung (4), die die in der Folie (11) enthaltene Flüssigkeitsmenge von der Position eines vorderen Endteils der Folie aus anzeigt, an einem Außenumfang des zylindrischen Körpers (2) vorgesehen ist.

7. Eine Vorrichtung gemäß einem beliebigen der vorstehenden Ansprüche, in der eine Feder (10) in einer Spiralform, die am Außenumfang der gummi-elastischen Folie (11) positioniert ist und sich mit der Ausdehnung der Folie ausdehnt, im Deckelkörper (5) in Eingriff steht.

8. Eine Vorrichtung gemäß Anspruch 7, in der die Feder (10) spiralförmig gewendelt ist, wobei der Durchmesser in Richtung weg vom Deckelkörper immer kleiner wird, ein Ende im Deckelkörper (5) in Eingriff steht und das andere Ende an das vordere Ende des Einführungsrohrs (7) für die medizinische Flüssigkeit anliegt und die Folie (11) zwischen ihnen liegt.

9. Eine Vorrichtung gemäß einem beliebigen der vorstehenden Ansprüche, in der ein Luftloch (9) im Deckelkörper (5) ausgebildet ist und ein wasserabstoßender Luftfilter (9A) innerhalb des Luftlochs befestigt ist.

10. Eine Vorrichtung gemäß einem beliebigen der vorstehenden Ansprüche, in der das Rückschlagventil (13) mit einer Ventilhülse (15) versehen ist, die innerhalb des Flüssigkeitszufuhrlochs (19) eingelegt ist und einen Ventilsitz (14) in einer Mittelstellung drin aufweist, und eine Ventilstange (16) in der Ventilhülse enthalten ist, so daß sie sich frei bewegen kann und den Ventilsitz öffnet und schließt.

11. Eine Vorrichtung gemäß Anspruch 10, in der eine Abdeckung (17) an einem Außenende der Ventilhülse (15) so vorgesehen ist, daß sie sich frei bewegen kann und daß sie eingerastet werden kann.

12. Eine Vorrichtung gemäß einem beliebigen der vorstehenden Ansprüche, in der das Flüssigkeitszufuhrrohr (24) durch ein Thermoplastharzrohr mit einer Öffnung mit nichtkreisförmigem Querschnitt und mit einer vorgegebenen Länge angeordnet ist.

## Revendications

1. Dispositif d'alimentation en fluide comprenant un boîtier de protection (1), un film élastique en caoutchouc tubulaire (11) qui est reçu dans le boîtier de protection et est agencé de manière à contenir un fluide, et un tube d'entrée de fluide (24) à travers lequel le fluide disposé à l'intérieur du film est fourni sous l'effet de la force de contraction du film, dans lequel le boîtier de protection est agencé sous la forme d'un boîtier cylindrique (2) ayant un fond et un corps de couvercle (5) en prise dans une extrémité ouverte du corps de cylindre, et dans lequel le boîtier de protection est réalisé dans le corps de couvercle, avec un orifice de fluide d'entrée (19) à travers lequel le fluide est introduit dans le film, un orifice de fluide de sortie (18) à travers lequel le fluide s'écoule dans le tube d'alimentation, et un tube d'introduction de fluide (7) inséré dans le film, dont le diamètre extérieur et la longueur sont pratiquement égaux au diamètre extérieur et à la longueur du film à l'état contracté et qui est pourvu de fentes (8) sur une surface circonférentielle de celui-ci grâce auxquelles l'intérieur et l'extérieur du tube d'introduction de fluide sont en communication fluidique l'un avec l'autre ; caractérisé par le fait que l'extrémité intérieure de l'orifice de fluide d'entrée (19) et l'extrémité de l'orifice de fluide de sortie (18) sont réunies de sorte que les deux orifices de fluide communiquent avec le tube d'introduction de fluide (7) et le film élastique à l'état de caoutchouc (11) à travers les fentes (8) ; par le fait que l'extrémité extérieure de l'orifice de fluide d'entrée et de l'orifice de fluide de sortie s'étendent en s'éloignant l'une de l'autre par rapport à l'axe principal du tube d'introduction de fluide sous une forme en Y ; et par le fait qu'un clapet anti-retour (13) qui permet l'entrée du fluide et empêche la sortie du fluide est présent au niveau de l'orifice de fluide d'entrée (19).

2. Dispositif selon la revendication 1, dans lequel le film est un tube pourvu d'un fond ayant une extrémité ouverte, et dans lequel une partie ouverte d'une extrémité est fixée à la partie de base du tube d'introduction de fluide décrite ci-dessus, à l'aide d'outils de fixation.

3. Dispositif selon l'une quelconque des revendications précédentes, dans lequel le corps cylindrique (2) présente une section transversale elliptique.

4. Dispositif selon l'une quelconque des revendications précédentes, dans lequel le fond du corps cylindrique (2) est réalisé de façon à avoir une forme convexe incurvée.

5. Dispositif selon l'une quelconque des revendications précédentes, dans lequel le corps cylindrique (2) est constitué d'un matériau transparent.

6. Dispositif selon l'une quelconque des revendications précédentes, dans lequel une division (4) représentant la quantité de fluide contenue dans le film (11) à partir de la position de la partie d'extrémité avant du film est réalisée sur la circonférence extérieure du corps cylindrique (2).

7. Dispositif selon l'une quelconque des revendications précédentes, dans lequel un ressort (10) de forme spiralée qui est placé sur la circonférence extérieure du film élastique à l'état de caoutchouc (11) et qui s'allonge avec l'allongement du film, vient en prise dans le corps de couvercle (5).

8. Dispositif selon la revendication 7, dans lequel le ressort (10) est enroulé sous la forme d'une spirale dans laquelle le diamètre devient plus étroit en s'éloignant du corps de couvercle, l'une de ses extrémités venant en prise dans le corps de couvercle (5) et l'autre extrémité venant en butée contre l'extrémité avant du tube d'introduction de fluide médical (7), le film (11) étant placé entre elles.

9. Dispositif selon l'une quelconque des revendications précédentes, dans lequel un évent (9) est réalisé dans le corps de couvercle (5) et un filtre à air rejetant l'eau (9A) est fixé à l'intérieur de l'évent.

10. Dispositif selon l'une quelconque des revendications précédentes, dans lequel le clapet antiretour (13) comporte une gaine de clapet (15) qui est disposée à l'intérieur de l'orifice de fluide d'entrée (19) et comporte un siège de clapet (14) à mi-distance à l'intérieur de celui-ci, et une tige de clapet (16) disposée à l'intérieur de la gaine de clapet de manière à être libre de se mouvoir, ouvrant et fermant le siège de clapet.

11. Dispositif selon la revendication 10, dans lequel un capuchon (17) est présent à l'extrémité extérieure de la gaine de clapet (15) de manière à être libre de se mouvoir et a venir en prise.

12. Dispositif selon l'une quelconque des revendications précédentes, dans lequel le tube d'alimentation en fluide (24) est constitué par un tube en résine thermoplastique de forme ouverte en coupe non circulaire et une longueur prédéterminée.
